# EUROPÄISCHE PATENTANMELDUNG

(11) **EP 4 696 351 A1**
(43) Veröffentlichungstag der Anmeldung: **18.02.2026**
(21) Anmeldenummer: 25185464.2
(22) Anmeldetag: 26.06.2025
(51) Int. Cl.: A61M 16/00, A61M 16/10

(54) **STEUEREINHEIT FÜR EIN BEATMUNGSGERÄT**

(30) Priorität: 14.08.2024 DE 102024123174
(71) Anmelder: Löwenstein Medical Technology S.A., 2557 Luxembourg (LU)
(72) Erfinder: Kremeier, Peter, 76149 Karlsruhe (DE); Tusman, Gerardo, 7600 Mar del Plata (AR)
(74) Vertreter: Löwenstein Medical IP

(57) **Zusammenfassung**

Ein Beatmungsgerät (1) umfasst: einen Atemluftanschluss (3), an den der Atemapparat (15) eines Patienten angeschlossen ist, sodass eine Beatmung des Patienten mit Atemluft möglich ist; eine Aktorik (5) zum Bereitstellen eines Atemluftstroms (7) am Atemluftanschluss (3); eine Sensorik (9) zum Erzeugen von Messdaten (11) bezüglich der Beatmung. Eine Steuereinheit (13) für das Beatmungsgerät (1) ist konfiguriert, um folgendes Verfahren (M) auszuführen: Erzeugen eines Steuersignals (25) zum Steuern der Aktorik (5), sodass ein Beatmungsmanöver durchgeführt wird, bei dem zumindest ein Abschnitt (17, 19) des Atemapparats (15) in einer Einatmungsphase geöffnet und/oder in einer Ausatmungsphase verschlossen wird; Empfangen der Messdaten (11), wobei die Messdaten (11) einen druckabhängigen Verlauf einer vom Patienten in der Einatmungsphase eingeatmeten und/oder in der Ausatmungsphase ausgeatmeten Menge mindestens eines Atemgases abhängig von einem Druck der Atemluft anzeigen; Bestimmen eines Öffnungsdrucks, der einem Druck der Atemluft entspricht, bei dem zumindest ein Abschnitt (17, 19) des Atemapparats (15) geöffnet wird, durch Auswerten eines auf die Einatmungsphase bezogenen Abschnitts des druckabhängigen Verlaufs, und/oder Bestimmen eines Verschlussdrucks, der einem Druck der Atemluft entspricht, bei dem zumindest ein Abschnitt (17, 19) des Atemapparats (15) verschlossen wird, durch Auswerten eines auf die Ausatmungsphase bezogenen Abschnitts des druckabhängigen Verlaufs.

## Beschreibung

### Technisches Gebiet

Die Erfindung betrifft eine Steuereinheit für ein Beatmungsgerät. Des Weiteren betrifft die Erfindung ein durch die Steuereinheit ausführbares Computerprogramm, ein entsprechendes computerlesbares Medium und ein Beatmungsgerät mit einer solchen Steuereinheit.

### Stand der Technik

Bei künstlich beatmeten Patienten kann es beim Ausatmen unter bestimmten Umständen zu einem wiederkehrenden Kollaps der Lunge oder eines Teils der Lunge kommen. Schlimmstenfalls können dadurch lokale Verletzungen in der Lunge verursacht werden, die sich ungünstig auf die Prognose des Patienten auswirken können. Die Beatmung sollte daher so erfolgen, dass solche Lungenkollapse möglichst vermieden werden.

Darüber hinaus konnte gezeigt werden, dass es im Zusammenhang mit Manövern zur Lungenrekrutierung wichtig ist, nicht nur einen Kollaps der Lunge, sondern auch einen Verschluss der Atemwege zu erkennen.

Ein solcher Verschluss der Atemwege kann zur Unterbrechung des Atemluftflusses zwischen der proximalen Atemwegöffnung und den distalen (kleineren) Atemwegstrukturen und/oder den distalen Alveolarstrukturen führen. Infolgedessen kann ein erneutes Aufblähen der Lunge nur erfolgen, wenn die Atemwege wieder geöffnet werden. Ein dazu erforderlicher Druck kann auch als Atemwegöffnungsdruck bezeichnet werden. Der Druck in den Atemwegen kann jedoch deutlich vom Druck in den Alveolen abweichen - auch bei einem endexspiratorischen Verschluss. Dies kann zu Fehleinschätzungen der Beatmungssituation führen. Es besteht daher Bedarf an einer zuverlässigen und einfach zu implementierenden Methode zur Erkennung derartiger Drücke.

### Offenbarung der Erfindung

Eine Aufgabe der Erfindung kann darin gesehen werden, eine Steuereinheit bereitzustellen, die es ermöglicht, ein Öffnen und/oder Verschließen zumindest eines Abschnitts des Atemapparats eines Patienten bei dessen Beatmung in zuverlässiger Weise zu erkennen. Eine weitere Aufgabe der Erfindung kann darin gesehen werden, ein entsprechendes Computerprogramm, ein entsprechendes computerlesbares Medium und ein entsprechendes Beatmungsgerät bereitzustellen.

Diese Aufgaben werden durch die Gegenstände der unabhängigen Ansprüche gelöst. Vorteilhafte Ausführungsformen der Erfindung sind in den abhängigen Ansprüchen, der nachfolgenden Beschreibung und den beigefügten Figuren dargelegt.

Ein erster Aspekt der Erfindung betrifft eine Steuereinheit für ein Beatmungsgerät. Das Beatmungsgerät umfasst: einen Atemluftanschluss, an den der Atemapparat eines Patienten angeschlossen ist, sodass eine Beatmung des Patienten mit Atemluft möglich ist; eine Aktorik zum Bereitstellen eines Atemluftstroms am Atemluftanschluss; eine Sensorik zum Erzeugen von Messdaten bezüglich der Beatmung. Die Steuereinheit ist konfiguriert, um folgendes Verfahren auszuführen: Erzeugen eines Steuersignals zum Steuern der Aktorik, sodass ein Beatmungsmanöver durchgeführt wird, bei dem zumindest ein Abschnitt des Atemapparats in einer Einatmungsphase geöffnet und/oder in einer Ausatmungsphase verschlossen wird (oder kollabiert); Empfangen der Messdaten, wobei die Messdaten einen druckabhängigen Verlauf einer vom Patienten in der Einatmungsphase eingeatmeten und/oder in der Ausatmungsphase ausgeatmeten Menge mindestens eines Atemgases abhängig von einem Druck der Atemluft anzeigen; Bestimmen eines Öffnungsdrucks, der einem Druck der Atemluft entspricht, bei dem zumindest ein Abschnitt des Atemapparats geöffnet wird, durch Auswerten eines auf die Einatmungsphase bezogenen Abschnitts des druckabhängigen Verlaufs, und/oder Bestimmen eines Verschlussdrucks, der einem Druck der Atemluft entspricht, bei dem zumindest ein Abschnitt des Atemapparats verschlossen wird (oder kollabiert), durch Auswerten eines auf die Ausatmungsphase bezogenen Abschnitts des druckabhängigen Verlaufs.

In Versuchen hat sich überraschenderweise gezeigt, dass der Verlauf des Kohlendioxid- bzw. Sauerstoffpartialdrucks über dem Beatmungsdruck in einem entsprechenden (druckbasierten) Kapno- bzw. Oxigramm charakteristische Knickpunkte aufweist, die mit den Öffnungs- bzw. Verschlussdrücken, wie sie in einem herkömmlichen bettseitigen Lungenrekrutierungsmanöver mit anschließender PEEP-Titration bestimmt werden können, in signifikanter Weise korrelieren. Anders ausgedrückt können die Öffnungs- bzw. Verschlussdrücke direkt aus einem derartigen Kapno- bzw. Oxigramm abgelesen werden, ohne dass zwangsläufig zusätzliche Informationen zur Plausibilisierung herangezogen werden müssen. Dies stellt einen erheblichen Vorteil in Bezug auf Genauigkeit, Zuverlässigkeit und Effizienz gegenüber herkömmlichen Rekrutierungs- oder Titrationsmethoden dar.

Im Gegensatz zu komplexen wahrscheinlichkeitsbasierten Berechnungsverfahren zur Beurteilung des Atemwegöffnungsdrucks und der Lungenmechanik bei der Beatmung ermöglicht ein solches Verfahren eine genaue und zuverlässige Bestimmung des Öffnungs- bzw. Verschlussdrucks mit deutlich verringertem Rechenaufwand. Ein weiterer Vorteil ist, dass eine zeitaufwendige PEEP-Titration über mehrere Atemzüge entfallen kann. Zudem kann dadurch das Risiko einer Lungenüberdehnung, wie sie durch einen erhöhten Beatmungsdruck über einen längeren Zeitraum auftreten kann, verringert werden.

Die Steuereinheit kann Mittel zur Datenverarbeitung umfassen. Die Mittel zur Datenverarbeitung können als Hard- und/oder Software implementiert sein und/oder einen Prozessor umfassen. Der Prozessor kann konfiguriert sein, um das (computerimplementierte) Verfahren auszuführen. Zusätzlich zum Prozessor kann die Steuereinheit mindestens eines der folgenden Mittel zur Datenverarbeitung umfassen: einen Speicher, ein Bussystem zur Datenkommunikation zwischen dem Speicher und dem Prozessor, eine Datenkommunikationsschnittstelle zur drahtlosen und/oder drahtgebundenen Datenkommunikation mit Peripheriegeräten. Alternativ kann die Steuereinheit ausschließlich als Hardware, beispielsweise in Form eines ASIC- oder FPGA-Bausteins, implementiert sein.

Unter "Atemluft" kann ein einzelnes Atemgas oder ein Gemisch aus mehreren Atemgasen zum Beatmen des Patienten verstanden werden. Unter "Atemgas" kann beispielsweise Kohlendioxid, Sauerstoff, Stickstoff, Wasserdampf oder Narkosegas verstanden werden.

Unter "Beatmungsmanöver" kann allgemein ein Manöver verstanden werden, bei dem sich infolge eines entsprechend gesteuerten Drucks und/oder Volumenstroms der Atemluft zumindest ein Abschnitt des Atemapparats, beispielsweise zumindest ein Abschnitt der Atemwege und/oder der Lunge, genauer der Alveolen, beim Einatmen öffnet (auch Rekrutierung genannt) und/oder beim Ausatmen verschließt (oder kollabiert). Das Beatmungsmanöver kann während eines einzelnen Atemzugs oder während mehrerer, beispielsweise unmittelbar aufeinanderfolgender Atemzüge durchgeführt werden.

Der zumindest abschnittsweise Verschluss des Atemapparats ist so zu verstehen, dass der Verschluss nicht aktiv geschieht, sondern (rein) passiv aufgrund der Oberflächenspannung und der Rückstellkräfte des Lungengewebes und des Thorax. Diesem lawinenartigen System kann bei der Beatmung gezielt entgegengewirkt werden, beispielsweise derart, dass eine entsprechende Vergrößerung oder Verkleinerung des Zeitraums und/oder des Druckbereichs des Verschlusses bewirkt wird. Somit ist unter dem Begriff "Verschluss" insbesondere ein Kollabieren zu verstehen.

Es ist möglich, dass das Steuersignal unter zusätzlicher Verwendung der Messdaten (oder zumindest eines Teils der Messdaten) erzeugt wird. Dies ermöglicht es, das Beatmungsmanöver abhängig vom aktuellen Zustand des Patienten zu steuern.

Unter "Menge" kann ein Partialdruck oder eine Konzentration des jeweiligen Atemgases im Atemluftstrom und/oder im Blut des Patienten verstanden werden.

Die Messdaten können beim Durchführen des Beatmungsmanövers unter Verwendung der Sensorik erzeugt worden sein. Beispielsweise können die zum Bestimmen des Öffnungsdrucks verwendeten Messdaten, d. h. die Messdaten, die den auf die Einatmungsphase bezogenen Abschnitt des druckabhängigen Verlaufs anzeigen, in der Einatmungsphase des Beatmungsmanövers erzeugt worden sein. In entsprechender Weise können die zum Bestimmen des Verschlussdrucks verwendeten Messdaten, d. h. die Messdaten, die den auf die Ausatmungsphase bezogenen Abschnitt des druckabhängigen Verlaufs anzeigen, in der Ausatmungsphase des Beatmungsmanövers erzeugt worden sein. Zusätzlich kann zum Bestimmen des Öffnungsdrucks und/oder des Verschlussdrucks mindestens ein sonstiger Abschnitt des druckabhängigen Verlaufs ausgewertet werden.

Es ist möglich, dass die Messdaten während eines einzelnen Atemzugs erzeugt und/oder bei jedem Atemzug neu erzeugt und/oder neu empfangen werden. Anders ausgedrückt kann der druckabhängige Verlauf auf einen einzelnen Atemzug bezogen sein und/oder bei jedem Atemzug aktualisiert werden. Abhängig vom jeweiligen Atemgas kann der druckabhängige Verlauf beispielsweise auch als druckbasiertes oder barometrisches Kapno- oder Oxigramm bezeichnet werden.

Ein zweiter Aspekt der Erfindung betrifft ein Beatmungsgerät. Das Beatmungsgerät umfasst: einen Atemluftanschluss zum Anschließen des Atemapparats eines Patienten, sodass eine Beatmung des Patienten mit Atemluft möglich ist; eine Aktorik zum Bereitstellen eines Atemluftstroms am Atemluftanschluss; eine Sensorik zum Erzeugen von Messdaten bezüglich der Beatmung; eine Steuereinheit, wie sie vor- und nachstehend beschrieben wird.

Unter "Beatmungsgerät" kann beispielsweise ein Gerät zum invasiven und/oder nicht invasiven Beatmen des Patienten und/oder ein Anästhesiegerät verstanden werden.

Der Atemluftanschluss kann über einen oder mehrere Beatmungsschläuche und/oder über eine geeignete Patientenschnittstelle wie beispielsweise eine Maske, eine Nasenkanüle oder einen Tubus an den Atemapparat anschließbar sein.

Die Aktorik kann beispielsweise ein oder mehrere Gebläse und/oder ein oder mehrere elektrisch steuerbare Ventile umfassen.

Die Sensorik kann beispielsweise ausgebildet sein, um mindestens eine der folgenden Größen zu erfassen: die vom Patienten in der Einatmungsphase eingeatmete und/oder in der Ausatmungsphase ausgeatmete Menge des jeweiligen Atemgases, den Druck der Atemluft, ein Volumen der Atemluft, einen Volumenstrom der Atemluft. Die Sensorik kann einen oder mehrere Sensoren umfassen. Der Sensor kann bzw. zumindest einige der Sensoren können in einem Hauptstrom und/oder einem Nebenstrom der vom Patienten ein- und/oder ausgeatmeten Atemluft angeordnet sein.

Ein dritter Aspekt der Erfindung betrifft ein Computerprogramm zum Betreiben eines Beatmungsgeräts, wie es vor- und nachstehend beschrieben wird. Das Computerprogramm umfasst Befehle, die die Steuereinheit, beispielsweise einen Prozessor der Steuereinheit, beim Ausführen des Computerprogramms durch die Steuereinheit veranlassen, folgendes Verfahren auszuführen: Erzeugen eines Steuersignals zum Steuern der Aktorik, sodass ein Beatmungsmanöver durchgeführt wird, bei dem zumindest ein Abschnitt des Atemapparats in einer Einatmungsphase geöffnet und/oder in einer Ausatmungsphase verschlossen wird (oder kollabiert); Empfangen der Messdaten, wobei die Messdaten einen druckabhängigen Verlauf einer vom Patienten in der Einatmungsphase eingeatmeten und/oder in der Ausatmungsphase ausgeatmeten Menge mindestens eines Atemgases abhängig von einem Druck der Atemluft anzeigen; Bestimmen eines Öffnungsdrucks, der einem Druck der Atemluft entspricht, bei dem zumindest ein Abschnitt des Atemapparats geöffnet wird, durch Auswerten eines auf die Einatmungsphase bezogenen Abschnitts des druckabhängigen Verlaufs, und/oder Bestimmen eines Verschlussdrucks, der einem Druck der Atemluft entspricht, bei dem zumindest ein Abschnitt des Atemapparats verschlossen wird (oder kollabiert), durch Auswerten eines auf die Ausatmungsphase bezogenen Abschnitts des druckabhängigen Verlaufs.

Ein vierter Aspekt der Erfindung betrifft ein computerlesbares Medium, auf dem ein Computerprogramm, wie es vor- und nachstehend beschrieben wird, gespeichert ist.

Das computerlesbare Medium kann ein flüchtiger oder nicht flüchtiger Datenspeicher sein. Beispielsweise kann das computerlesbare Medium eine Festplatte, ein USB-Speichergerät (USB = *universal serial bus*), ein RAM (r*andom-access memory*), ein ROM (*read-only memory*), ein EPROM (*erasable programmable read-only memory*), ein EEPROM (*electrically erasable programmable read-only memory*), ein Flash-Speicher oder eine Kombination aus mindestens zwei dieser Beispiele sein. Das computerlesbare Medium kann auch ein Datenkommunikationsnetzwerk, das das Herunterladen von Programmcode ermöglicht (z. B. über das Internet), oder eine Cloud sein.

Es wird darauf hingewiesen, dass Merkmale der vor- und nachstehend beschriebenen Steuereinheit auch Merkmale des Computerprogramms und/oder des computerlesbaren Mediums sein können (und umgekehrt).

Im Folgenden werden verschiedene Ausführungsformen der Erfindung beschrieben. Diese Ausführungsformen sind nicht als Beschränkung des Umfangs der Erfindung zu verstehen.

Gemäß einer Ausführungsform kann der Öffnungsdruck einen Atemwegöffnungsdruck umfassen, der einem Druck der Atemluft entspricht, bei dem die (zuvor zumindest teilweise verschlossenen) Atemwege des Patienten zumindest teilweise geöffnet werden. Zusätzlich oder alternativ kann der Öffnungsdruck einen Lungenöffnungsdruck umfassen, der einem Druck der Atemluft entspricht, bei dem die (zuvor zumindest teilweise kollabierte) Lunge des Patienten zumindest teilweise geöffnet oder rekrutiert wird.

Gemäß einer Ausführungsform kann der Verschlussdruck einen Lungenverschlussdruck umfassen, der einem Druck der Atemluft entspricht, bei dem die (zuvor zumindest teilweise geöffnete oder rekrutierte) Lunge des Patienten zumindest teilweise verschlossen wird, d. h. zumindest teilweise kollabiert. Zusätzlich oder alternativ kann der Verschlussdruck einen Atemwegverschlussdruck umfassen, der einem Druck der Atemluft entspricht, bei dem die (zuvor zumindest teilweise geöffneten) Atemwege des Patienten zumindest teilweise verschlossen werden (oder zumindest teilweise kollabieren). Gemäß einer Ausführungsform kann das Steuersignal so erzeugt werden, dass ein Einatmungsdruck, der einem Druck der Atemluft in der Einatmungsphase entspricht, in mehreren aufeinanderfolgenden Zeitschritten erhöht wird. Dabei kann der Einatmungsdruck in jedem Zeitschritt höher als im jeweils vorangegangenen Zeitschritt sein. Anders ausgedrückt ist es möglich, dass der Einatmungsdruck ausgehend von einem vorgegebenen Anfangswert bis zu einem vorgegebenen Endwert stetig erhöht wird. Zusätzlich oder alternativ kann der Einatmungsdruck einem vorgegebenen Einatmungsdruckverlauf folgen. Dabei kann der Einatmungsdruckverlauf zumindest teilweise linear ansteigen. Es ist zweckmäßig, wenn der Anfangswert einem Einatmungsdruck entspricht, bei dem zumindest ein Abschnitt des Atemapparats verschlossen ist. Beispielsweise kann der Anfangswert des Einatmungsdrucks null sein oder dem jeweiligen Umgebungsdruck entsprechen. Hingegen sollte der Endwert einem Einatmungsdruck entsprechen, bei dem die Lunge zumindest größtenteils geöffnet oder rekrutiert ist, ohne übermäßig gedehnt zu werden. Beispielsweise kann der Endwert des Einatmungsdrucks höchstens 30 cmH₂O, höchstens 40 cmH₂O, höchstens 50 cmH₂O oder höchstens 60 cmH₂O betragen. Je nach Zustand des jeweiligen Patienten sind aber auch andere Anfangs- und/oder Endwerte des Einatmungsdrucks möglich.

Gemäß einer Ausführungsform kann das Steuersignal so erzeugt werden, dass ein Volumenstrom der Atemluft in der Ausatmungsphase einem vorgegebenen (beispielsweise zeitabhängigen) Volumenstromverlauf folgt. Anders ausgedrückt kann die Beatmung in der Ausatmungsphase flussgesteuert sein. Der Volumenstromverlauf kann zumindest teilweise konstant sein und/oder zumindest teilweise ansteigen und/oder zumindest teilweise abfallen. Dies ermöglicht eine genauere Auswertung des druckabhängigen Verlaufs im Vergleich zu einer druckgesteuerten Ausatmungsphase.

Gemäß einer Ausführungsform kann ein Volumenstrom der Atemluft in der Ausatmungsphase zwischen 0,02 l/s und 0,20 I/s, bevorzugt zwischen 0,05 l/s und 0,15 I/s, besonders bevorzugt bei 0,10 l/s, liegen. Alternativ kann der Volumenstrom mindestens 0,02 I/s, bevorzugt mindestens 0,05 I/s, und/oder höchstens 0,20 I/s, bevorzugt höchstens 0,15 I/s, betragen. Dies ermöglicht aufgrund des im Vergleich zu einer druckgesteuerten Ausatmungsphase deutlich vergrößerten Zeitfensters eine besonders genaue Auswertung des druckabhängigen Verlaufs.

Gemäß einer Ausführungsform kann das Steuersignal so erzeugt werden, dass ein Ausatmungsdruck, der einem Druck der Atemluft in der Ausatmungsphase entspricht, in mehreren aufeinanderfolgenden Zeitschritten verringert wird. Dabei kann der Ausatmungsdruck in jedem Zeitschritt niedriger als im jeweils vorangegangenen Zeitschritt sein. Anders ausgedrückt ist es möglich, dass der Ausatmungsdruck ausgehend von einem vorgegebenen Anfangswert bis zu einem vorgegebenen Endwert stetig verringert wird. Zusätzlich oder alternativ kann der Ausatmungsdruck einem vorgegebenen Ausatmungsdruckverlauf folgen. Dabei kann der Ausatmungsdruckverlauf zumindest teilweise linear abfallen. Der Endwert des Ausatmungsdrucks kann beispielsweise null betragen oder dem jeweiligen Umgebungsdruck entsprechen. Es ist möglich, dass der Anfangswert des Ausatmungsdrucks mit einem Endwert eines Einatmungsdrucks am Ende einer vorangegangenen Einatmungsphase übereinstimmt und/oder dass der Endwert des Ausatmungsdrucks mit einem Anfangswert eines Einatmungsdrucks am Anfang einer nachfolgenden Einatmungsphase übereinstimmt. Der Endwert des Ausatmungsdrucks kann beispielsweise auch als positiver endexspiratorischer Druck, kurz PEEP, bezeichnet werden. Dementsprechend kann der Anfangswert des Einatmungsdrucks dem PEEP eines vorangegangenen Atemzugs entsprechen.

Gemäß einer Ausführungsform kann das Bestimmen des Öffnungsdrucks und/oder des Verschlussdrucks umfassen: Erkennen eines charakteristischen Knickpunkts im druckabhängigen Verlauf; Bestimmen eines dem charakteristischen Knickpunkt zugeordneten Drucks der Atemluft, um den Öffnungsdruck und/oder den Verschlussdruck zu bestimmen. Anders ausgedrückt kann das Bestimmen des Öffnungsdrucks und/oder des Verschlussdrucks ein Erkennen einer charakteristischen Änderung einer Steigung des druckabhängigen Verlaufs umfassen.

Sind die Atemwege verschlossen und erreicht der Einatmungsdruck eine kritische Schwelle, so öffnen sich die Atemwege schlagartig, sodass frische, d. h. sauerstoffreiche Atemluft in die Lunge strömen kann. Dadurch kommt es zur Verdünnung der verbrauchten, d. h. kohlendioxidreichen Atemluft, die beim vorherigen Ausatmen im Atemapparat verblieben ist, was sich in einem entsprechend starken Abfall des Kohlendioxidpartialdrucks im Atemluftstrom äußert. Der Einfluss dieser plötzlichen Eröffnung ist in einem entsprechenden druckbasierten Kapnogramm beispielsweise als ein charakteristischer Knickpunkt zu erkennen. Der Druck der Atemluft, der diesem Knickpunkt zugeordnet ist, kann somit als der Atemwegöffnungsdruck betrachtet werden.

Gemäß einer Ausführungsform kann der charakteristische Knickpunkt anhand einer charakteristischen betragsmäßigen Verringerung einer beispielsweise durchschnittlichen Steigung des druckabhängigen Verlaufs mit zunehmender Beatmungsdauer erkannt werden. Dies ermöglicht auch bei einem stark schwankenden Zustand des Patienten eine genaue und zuverlässige Erkennung des Öffnungs- bzw. Verschlussdrucks.

Der dem Öffnungsdruck, insbesondere dem Atemwegöffnungsdruck, zugeordnete charakteristische Knickpunkt kann beispielsweise einer betragsmäßigen Verringerung der (durchschnittlichen) Steigung des druckabhängigen Verlaufs um mindestens 50 Prozent oder um mindestens 70 Prozent im ersten Drittel der Einatmungsphase entsprechen.

Beispielsweise kann zum Bestimmen des Öffnungsdrucks ein Abschnitt des druckabhängigen Verlaufs über einem Einatmungsdruckbereich zwischen 0 und 20 cmH₂O oder zwischen 5 und 15 cmH₂O oder zwischen 10 und 15 cmH₂O ausgewertet werden. Je nach Zustand des jeweiligen Patienten sind aber auch andere Einatmungsdruckbereiche möglich.

Beispielsweise kann zum Bestimmen des Verschlussdrucks ein Abschnitt des druckabhängigen Verlaufs über einem Ausatmungsdruckbereich zwischen 0 und 20 cmH₂O oder zwischen 0 und 15 cmH₂O oder zwischen 0 und 10 cmH₂O ausgewertet wird. Je nach Zustand des jeweiligen Patienten sind aber auch andere Ausatmungsdruckbereiche möglich.

Gemäß einer Ausführungsform können die Einatmungsphase und die Ausatmungsphase aufeinanderfolgende Phasen eines einzelnen Atemzugs sein. Anders ausgedrückt kann der druckabhängige Verlauf auf einen einzelnen Atemzug bezogen sein.

Gemäß einer Ausführungsform kann zum Bestimmen des Öffnungsdrucks ein Abschnitt des druckabhängigen Verlaufs in der ersten Hälfte oder im ersten Drittel der Einatmungsphase (beispielsweise eines einzelnen Atemzugs) ausgewertet werden. Anders ausgedrückt kann der Öffnungsdruck durch Auswerten des druckabhängigen Verlaufs eher am Anfang als am Ende der Einatmungsphase bestimmt werden. Zusätzlich oder alternativ kann zum Bestimmen des Verschlussdrucks ein Abschnitt des druckabhängigen Verlaufs im letzten Drittel der Ausatmungsphase (beispielsweise im gleichen Atemzug wie die Einatmungsphase) ausgewertet werden. Anders ausgedrückt kann der Verschlussdruck durch Auswerten des druckabhängigen Verlaufs eher am Ende als am Anfang der Ausatmungsphase bestimmt werden.

Die Unterteilung der Ausatmungsphase in mehrere Phasenabschnitte kann beispielsweise durch Auswerten eines entsprechenden volumetrischen Kapno- oder Oxigramms erfolgen. Dabei kann der jeweilige volumenabhängige Verlauf beispielsweise in mindestens drei aufeinanderfolgende Phasenabschnitte, die für einen einzelnen Atemzug charakteristisch sind, unterteilt werden. Die Phasenabschnitte können sich signifikant in ihrer Länge und/oder in der (beispielsweise durchschnittlichen) Steigung des volumenabhängigen Verlaufs voneinander unterscheiden.

Beispielsweise kann im Fall eines volumetrischen Kapnogramms ein erster Phasenabschnitt vom Beginn der Ausatmung bis zu einem ersten Punkt reichen, an dem die Änderungsrate der zweiten Ableitung des volumenabhängigen Verlaufs ihr Maximum erreicht bzw. die dritte Ableitung des volumenabhängigen Verlaufs ihr linksseitiges Maximum erreicht. Ein zweiter Phasenabschnitt kann vom ersten Punkt bis zu einem zweiten Punkt reichen, an dem die dritte Ableitung des volumenabhängigen Verlaufs ihr rechtsseitiges Maximum erreicht. Ein dritter Phasenabschnitt kann vom zweiten Punkt bis zum Ende der Ausatmung reichen. Unter dem Begriff "volumenabhängiger Verlauf" kann hier auch eine geeignete Approximation verstanden werden. Beim ersten Phasenabschnitt kann es sich um die früheste Phase der Ausatmung (beispielsweise 10 % bis 12 % des gesamten Atemzugs) handeln, in der kaum oder kein Kohlendioxid in der Atemluft enthalten ist. Beim zweiten Phasenabschnitt kann es sich um eine Phase des größten (durchschnittlichen) Anstiegs der Kohlendioxidmenge im Atemluftstrom handeln (beispielsweise 15 % bis 18 % des gesamten Atemzugs). Beim dritten Phasenabschnitt kann es sich um eine Phase handeln, in der die Kohlendioxidmenge - im Gegensatz zu den vorangehenden Phasenabschnitten - überwiegend durch die aus den Alveolen kommenden Gase bestimmt wird (beispielsweise 70 % bis 75 % des gesamten Atemzugs).

Die Phasenabschnitte können beispielsweise entsprechend der Fowler'schen Methode und/oder dem Levenberg-Marquardt-Algorithmus näherungsweise aus dem (beispielsweise gemessenen) volumenabhängigen Verlauf bestimmt werden. Unter "Levenberg-Marquardt-Algorithmus" kann ein spezieller numerischer Optimierungsalgorithmus zur Lösung nicht linearer Ausgleichsprobleme mithilfe der Methode der kleinsten Quadrate verstanden werden. Der Algorithmus kann als eine Kombination des Gauß-Newton-Verfahrens mit einer Regularisierungstechnik, die absteigende Funktionswerte erzwingt, aufgefasst werden. Dies ermöglicht eine genauere und recheneffizientere Approximation als eine Implementierung der klassischen Fowler'schen Methode, und zwar auch bei stärkeren Schwankungen des volumenabhängigen Verlaufs zwischen aufeinanderfolgenden Atemzügen und/oder zwischen verschiedenen Patienten.

Alternativ können die Phasenabschnitte in entsprechender Weise anhand eines volumetrischen Oxigramms bestimmt werden.

Gemäß einer Ausführungsform können die Messdaten ferner einen volumenabhängigen Verlauf der vom Patienten in der Einatmungsphase eingeatmeten und/oder in der Ausatmungsphase ausgeatmeten Menge des mindestens einen Atemgases abhängig von einem Volumen der Atemluft anzeigen. Dabei kann der Öffnungsdruck ferner durch Auswerten eines auf die Einatmungsphase bezogenen Abschnitts des volumenabhängigen Verlaufs bestimmt werden. Zusätzlich oder alternativ kann der Verschlussdruck ferner durch Auswerten eines auf die Ausatmungsphase bezogenen Abschnitts des volumenabhängigen Verlaufs bestimmt werden. Beispielsweise kann jedem Punkt des volumenabhängigen Verlaufs ein bestimmter Anteil eines bei einem einzelnen Atemzug vom Patienten ein- und/oder ausgeatmeten Gesamtvolumens der (das jeweilige Atemgas umfassenden) Atemluft zugeordnet sein. Dementsprechend kann dem Anfang des volumenabhängigen Verlaufs ein Volumenwert von null und dem Ende des volumenabhängigen Verlaufs ein Volumenwert gleich dem Gesamtvolumen zugeordnet sein. Ein solches Gesamtvolumen kann auch als Atemzug- oder Tidalvolumen bezeichnet werden. Abhängig vom jeweiligen Atemgas kann der volumenabhängige Verlauf beispielsweise auch als volumenbasiertes oder volumetrisches Kapno- oder Oxigramm bezeichnet werden. Diese Ausführungsform ermöglicht eine zusätzliche Plausibilisierung der durch Auswerten des druckabhängigen Verlaufs erhaltenen Ergebnisse.

Gemäß einer Ausführungsform können die Messdaten ferner einen zeitabhängigen Verlauf eines vom Patienten in der Einatmungsphase eingeatmeten und/oder in der Ausatmungsphase ausgeatmeten Volumenstroms der Atemluft abhängig von einer Beatmungsdauer anzeigen. Dabei kann der Öffnungsdruck ferner durch Auswerten eines auf die Einatmungsphase bezogenen Abschnitts des zeitabhängigen Verlaufs bestimmt werden. Zusätzlich oder alternativ kann der Verschlussdruck ferner durch Auswerten eines auf die Ausatmungsphase bezogenen Abschnitts des zeitabhängigen Verlaufs bestimmt werden. In diesem Fall kann das Bestimmen des Öffnungsdrucks und/oder des Verschlussdrucks - ähnlich wie beim Auswerten des druckabhängigen Verlaufs - umfassen: Erkennen eines weiteren charakteristischen Knickpunkts im zeitabhängigen Verlauf; Bestimmen eines dem weiteren charakteristischen Knickpunkt zugeordneten Drucks der Atemluft, um den Öffnungsdruck und/oder den Verschlussdruck zu bestimmen. Diese Ausführungsform ermöglicht eine zusätzliche Plausibilisierung der durch Auswerten des druckabhängigen Verlaufs erhaltenen Ergebnisse.

Bei einem (vollständigen) Verschluss der Atemwege zu Beginn der Einatmungsphase steigt der Druck der Atemluft an, während der Volumenstrom der Atemluft gegen null geht. Erst wenn der Druck der Atemluft so hoch ist, dass sich die Atemwege öffnen, beginnt auch der Volumenstrom anzusteigen. Der Druck der Atemluft, der diesem ersten Knickpunkt im zeitabhängigen Verlauf des Volumenstroms in der Einatmungsphase zugeordnet ist, kann somit als der Atemwegöffnungsdruck betrachtet werden.

Nachdem die Atemwege geöffnet wurden, füllt sich die Lunge zunehmend mit Atemluft. Zu diesem Zeitpunkt können einige distale Abschnitte der Lunge noch kollabiert sein. Erst wenn der Druck der Atemluft groß genug ist, öffnen sich auch diese noch kollabierten Abschnitte wie beispielsweise die kleineren Atemweg- und Alveolarstrukturen. Dabei steigt der Volumenstrom der Atemluft entsprechend deutlich an. Der Druck der Atemluft, bei dem der Volumenstrom der Atemluft sein (lokales) Maximum in der Einatmungsphase erreicht, kann somit als der Lungenöffnungsdruck betrachtet werden. Wird der Lungenöffnungsdruck im weiteren Verlauf der Einatmungsphase überschritten, so fällt der Volumenstrom wieder deutlich ab, weil das Lungengewebe dann seine Elastizitätsgrenze erreicht hat. Bei einem weiteren Druckanstieg über diesen Punkt hinaus kann es zu einer unerwünschten Überdehnung der Lunge kommen.

Gemäß einer Ausführungsform kann das Verfahren ferner umfassen: Bestimmen mindestens eines Sollwerts für den Druck der Atemluft unter Berücksichtigung des Öffnungsdrucks und/oder des Verschlussdrucks; Verwenden des mindestens einen Sollwerts zum Steuern der Aktorik. Der Sollwert kann bzw. die Sollwerte können beispielsweise einen positiven endexspiratorischen Druck, kurz PEEP, vorgeben. Der Sollwert kann bzw. die Sollwerte können beispielsweise zwischen dem Öffnungsdruck und dem Verschlussdruck liegen. Somit kann eine zeitaufwendige Titration des individuellen PEEP für den jeweiligen Patienten, wie sie üblicherweise durchgeführt wird, entfallen.

Gemäß einer Ausführungsform können die Messdaten (beispielsweise gemessene und/oder geschätzte) Werte für mindestens eine der folgenden Größen umfassen und/oder auf (beispielsweise gemessenen und/oder geschätzten) Werten für mindestens eine der folgenden Größen basieren: die vom Patienten in der Einatmungsphase eingeatmete und/oder in der Ausatmungsphase ausgeatmete Menge des mindestens einen Atemgases, den Druck der Atemluft, ein Volumen der Atemluft, einen Volumenstrom der Atemluft. Das Volumen der Atemluft kann beispielsweise auch durch Integration des Volumenstroms der Atemluft bestimmt werden.

### Kurze Beschreibung der Zeichnungen

Im Folgenden werden Ausführungsformen der Erfindung mit Bezug auf die beigefügten Zeichnungen beschrieben. Weder die Beschreibung noch die Zeichnungen sind als Beschränkung des Umfangs der Erfindung zu verstehen.
Fig. 1 zeigt ein Beatmungsgerät gemäß einer Ausführungsform der Erfindung.
Fig. 2 zeigt ein Flussdiagramm zur Veranschaulichung eines Verfahrens, das durch eine Steuereinheit gemäß einer Ausführungsform der Erfindung ausgeführt werden kann.
Fig. 3 zeigt einen zeitabhängigen Druckverlauf und einen zeitabhängigen Volumenstromverlauf beim Durchführen eines Beatmungsmanövers unter Verwendung einer Steuereinheit gemäß einer Ausführungsform der Erfindung.
Fig. 4 zeigt ein druckbasiertes Kapnogramm zur Auswertung durch eine Steuereinheit gemäß einer Ausführungsform der Erfindung.
Fig. 5 zeigt ein volumenbasiertes Kapnogramm zur Auswertung durch eine Steuereinheit gemäß einer Ausführungsform der Erfindung.

Die Figuren sind rein schematisch und nicht maßstabsgetreu. Werden in verschiedenen Zeichnungen gleiche Bezugszeichen verwendet, so bezeichnen diese Bezugszeichen gleiche oder gleichwirkende Merkmale.

### Ausführungsformen der Erfindung

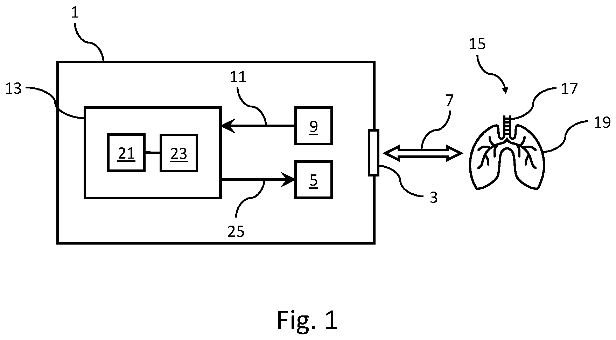

Fig. 1 zeigt ein Beatmungsgerät 1, das einen Atemluftanschluss 3, eine Aktorik 5 zum Bereitstellen eines Atemluftstroms 7 am Atemluftanschluss 3 und eine Sensorik 9 zum Erzeugen von Messdaten 11 in Bezug auf mindestens eine beatmungsrelevante Messgröße umfasst. Des Weiteren umfasst das Beatmungsgerät 1 eine mit der Aktorik 5 und der Sensorik 9 verbundene Steuereinheit 13 zum Steuern der Aktorik 5, beispielsweise unter zusätzlicher Verwendung der Messdaten 11.

Die Aktorik 5 kann ein oder mehrere Gebläse und/oder ein oder mehrere elektrische steuerbare Ventile umfassen.

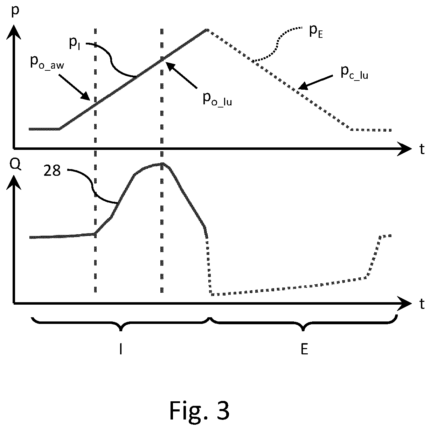

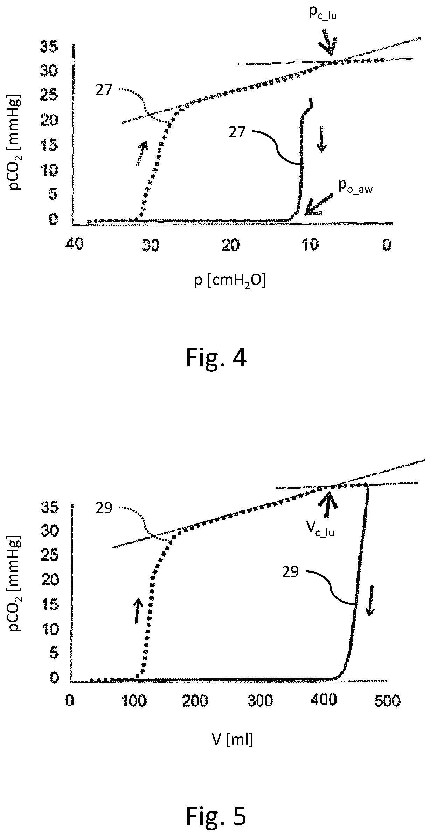

Die Sensorik 9 kann einen oder mehrere Sensoren umfassen, beispielsweise mindestens einen der folgenden Sensoren: einen Kohlendioxidsensor zum Erfassen eines Kohlendioxidpartialdrucks pCO₂ (siehe Fig. 4 und Fig. 5), einen Sauerstoffsensor zum Erfassen eines Sauerstoffpartialdrucks, einen Flusssensor zum Erfassen eines Volumenstroms Q der Atemluft, einen Drucksensor zum Erfassen eines Drucks p der Atemluft (siehe Fig. 3).

Der Atemluftanschluss 3 ist - beispielsweise über einen oder mehrere Beatmungsschläuche und eine geeignete Patientenschnittstelle wie beispielsweise eine Maske, eine Nasenkanüle oder einen Tubus - an den Atemapparat 15 eines Patienten angeschlossen, insbesondere an dessen Atemwege 17 und/oder dessen Lunge 19, sodass der Patient mit Atemluft beatmet werden kann. Je nach Anwendungsfall kann die Beatmung invasiv oder nicht invasiv erfolgen.

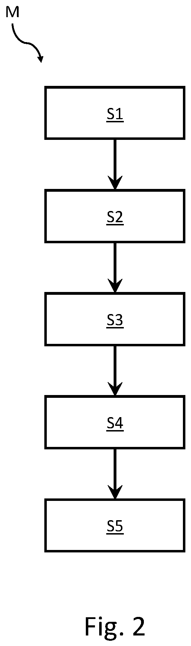

Die Steuereinheit 13 kann einen Prozessor 21 und einen Speicher 23 umfassen, in dem ein Computerprogramm zum Betreiben des Beatmungsgeräts 1 gespeichert sein kann. Der Prozessor 21 kann konfiguriert sein, um durch Ausführen des Computerprogramms folgendes Verfahren M zum Betreiben des Beatmungsgeräts 1 auszuführen. Der Ablauf des Verfahrens M ist in Fig. 2 veranschaulicht.

In einem Schritt S1 wird ein Steuersignal 25 zum Steuern der Aktorik 5 erzeugt, sodass ein spezielles Beatmungsmanöver, beispielsweise zum Rekrutieren der Lunge 19, durchgeführt wird. Wie an dem in Fig. 3 gezeigten Verlauf des Drucks p der Atemluft beim Beatmen des Patienten zu erkennen, kann das Beatmungsmanöver so durchgeführt werden, dass in einer Einatmungsphase I eines einzelnen Atemzugs zunächst ein Öffnen der (zuvor verschlossenen) Atemwege 17 und anschließend ein Öffnen der (zuvor zumindest teilweise kollabierten) Lunge 19 erfolgt. Darauf erfolgt in einer Ausatmungsphase E des gleichen Atemzugs erneut ein Verschließen, d. h. ein zumindest teilweises Kollabieren der Lunge 19. Das Beatmungsmanöver kann beispielsweise eine Minute oder kürzer dauern.

In einem Schritt S2 werden die Messdaten 11 empfangen. Die Messdaten 11 können beim Durchführen des Beatmungsmanövers erzeugt worden sein. In diesem Beispiel zeigen die Messdaten 11 einen druckabhängigen Verlauf 27 des Kohlendioxidpartialdrucks pCO₂, im Folgenden kurz druckabhängiger Verlauf 27 genannt, abhängig vom Druck p der vom Patienten beim Durchführen des Beatmungsmanövers geatmeten Atemluft an (siehe Fig. 4). Die Messdaten 11 können beispielsweise (gemessene und/oder geschätzte) Werte für mindestens eine der folgenden Messgrößen umfassen und/oder auf (gemessenen und/oder geschätzten) Werten für mindestens eine der folgenden Messgrößen basieren: den Kohlendioxidpartialdruck pCO₂, den Sauerstoffpartialdruck, den Druck p der Atemluft, ein Volumen V der Atemluft, einen Volumenstrom Q der Atemluft.

In einem Schritt S3 wird beispielsweise durch Auswerten des druckabhängigen Verlaufs 27 in der Einatmungsphase I (in Fig. 4 mit einer durchgehenden Linie angedeutet), insbesondere in der ersten Hälfte oder dem ersten Drittel der Einatmungsphase I, ein Atemwegöffnungsdruck p_{o_aw} bestimmt. Der Atemwegöffnungsdruck p_{o_aw} entspricht einem Druck p der Atemluft, bei dem ein (vollständiges) Öffnen der (beispielsweise beim vorherigen Ausatmen verschlossenen) Atemwege 17 erfolgt.

Zusätzlich oder alternativ kann durch Auswerten des druckabhängigen Verlaufs 27 in der Ausatmungsphase E (in Fig. 4 mit einer gestrichelten Linie angedeutet), insbesondere im letzten Drittel der Ausatmungsphase E, ein Lungenverschlussdruck p_{c_lu} bestimmt werden, der einem Druck p der Atemluft entspricht, bei dem die (beispielsweise beim vorherigen Einatmen geöffnete) Lunge 19 wieder zu kollabieren beginnt.

Wie in Fig. 3 gezeigt, kann ferner ein Lungenöffnungsdruck p_{o_lu} durch Auswerten eines zeitabhängigen Verlaufs 28 des Volumenstroms Q der Atemluft in der Einatmungsphase I, beispielsweise unter Verwendung der Messdaten 11, bestimmt werden. Der Lungenöffnungsdruck p_{o_lu} kann einem Druck p der Atemluft entsprechen, bei dem der Volumenstrom Q der Atemluft infolge des (vollständigen) Öffnens der (beispielsweise beim vorherigen Ausatmen zumindest teilweise kollabierten) Lunge 19 sein Maximum in der Einatmungsphase I erreicht.

Zweckmäßigerweise kann das Steuersignal 25 so erzeugt werden, dass sich ein Einatmungsdruck pₗ, der einem Druck p der Atemluft in der Einatmungsphase I entspricht, in mehreren aufeinanderfolgenden Zeitschritten erhöht. Dabei kann der Einatmungsdruck p_{I} in jedem Zeitschritt höher als im jeweils vorangegangenen Zeitschritt sein, sodass ein stetig ansteigender Verlauf des Einatmungsdrucks p_{I} resultiert. Zusätzlich kann die Aktorik 5 mittels des Steuersignals 25 gemäß einem vorgegebenen Einatmungsdruckverlauf so gesteuert werden, dass der Einatmungsdruck p_{I} zumindest größtenteils linear ansteigt. Möglich ist je nach Zustand des jeweiligen Patienten auch ein zumindest teilweise nicht linearer Verlauf des Einatmungsdrucks p_{I}.

Analog zum Einatmungsdruck p_{I} kann der Druck p der Atemluft in der Ausatmungsphase E mittels des Steuersignals 25 so gesteuert werden, dass ein stetig und/oder zumindest größtenteils linear abfallender Verlauf eines Ausatmungsdrucks p_{E} resultiert.

Um die Genauigkeit beim Auswerten des druckabhängigen Verlaufs 27 in der Ausatmungsphase E zu erhöhen, kann der Atemluftstrom 7 in der Ausatmungsphase E auch flussgesteuert bereitgestellt werden. Dazu kann das Steuersignal 25 so erzeugt werden, dass der Volumenstrom Q der Atemluft in der Ausatmungsphase E einem vorgegebenen, beispielsweise konstanten Volumenstromverlauf folgt. Geeignete Sollwerte für den Volumenstrom Q der Atemluft liegen beispielsweise zwischen 0,02 l/s und 0,20 I/s, bevorzugt zwischen 0,05 l/s und 0,15 I/s, besonders bevorzugt bei 0,10 I/s. Prinzipiell sollte der Volumenstrom Q in der Ausatmungsphase E nicht zu groß sein, damit ein ausreichend großes Zeitfenster zum Auswerten des druckabhängigen Verlaufs 27 zur Verfügung steht.

Wie in Fig. 4 am Beispiel des Lungenverschlussdrucks p_{c_lu} durch zwei an den druckabhängigen Verlauf 27 angelegte Steigungstangenten angedeutet, kann eine durchschnittliche Steigung des druckabhängigen Verlaufs 27 in mehreren aufeinanderfolgenden Zeitschritten ermittelt werden und anhand einer charakteristischen Verringerung dieser Steigung mit zunehmender Beatmungsdauer t ein charakteristischer Knickpunkt im druckabhängigen Verlauf 27 erkannt werden. Dieser Knickpunkt kann, je nachdem, in welchem Zeitabschnitt der Einatmungsphase I oder der Ausatmungsphase E er auftritt, den Atemwegöffnungsdruck p_{o_aw} oder den Lungenverschlussdruck p_{c_lu} anzeigen.

Sind die Atemwege 17 verschlossen und erreicht der Einatmungsdruck p_{I} eine kritische Schwelle, so öffnen sich die Atemwege 17 schlagartig, sodass frische, d. h. sauerstoffreiche Atemluft in die Lunge 19 strömen kann. Dadurch kommt es zur Verdünnung der verbrauchten, d. h. kohlendioxidreichen Atemluft, die beim vorherigen Ausatmen im Atemapparat 15 verblieben ist, was sich in einem entsprechend starken Abfall des Kohlendioxidpartialdrucks pCO₂ im Atemluftstrom 7 äußert. Der Druck p der Atemluft, der diesem Knickpunkt im druckabhängigen Verlauf 27 zugeordnet ist, kann als der Atemwegöffnungsdruck p_{o_aw} betrachtet werden.

Wie in Fig. 5 gezeigt, können die Messdaten 11 zusätzlich einen volumenabhängigen Verlauf 29 des Kohlendioxidpartialdrucks pCO₂, im Folgenden kurz volumenabhängiger Verlauf 29 genannt, abhängig vom Volumen V der Atemluft anzeigen. Der volumenabhängige Verlauf 29 kann beim Auswerten des druckabhängigen Verlaufs 27 zur zusätzlichen Plausibilisierung verwendet werden. Dazu kann beispielsweise anhand des volumenabhängigen Verlaufs 29 ein Lungenverschlussvolumen V_{c_lu} bestimmt werden, das einem Volumen V der Atemluft in der zumindest teilweise kollabierten Lunge 19 am oder gegen Ende der Ausatmungsphase E entspricht. Das Lungenverschlussvolumen V_{c_lu} und der Lungenverschlussdruck p_{c_lu} können zeitlich miteinander korrelieren, sodass sich die beiden Größen zur gegenseitigen Plausibilisierung eignen.

Darüber hinaus kann in einem Schritt S4 mindestens ein Sollwert für den Druck p der Atemluft unter Berücksichtigung des Atemwegöffnungsdrucks p_{o_aw} und/oder des Lungenöffnungsdrucks p_{o_lu} und/oder des Lungenverschlussdrucks p_{c_lu} bestimmt werden. Der mindestens eine Sollwert kann beispielsweise einen oberen Sollwert und einen unteren Sollwert umfassen, zwischen denen der Druck p der Atemluft variiert werden soll.

Schließlich kann der Sollwert bzw. können die Sollwerte in einem Schritt S5 zum weiteren Steuern der Aktorik 5 in einem normalen Beatmungsbetrieb, d. h. im Anschluss an das Beatmungsmanöver, verwendet werden.

Abschließend wird darauf hingewiesen, dass Begriffe wie "aufweisen", "umfassen", "einschließen", "mit" usw. keine anderen Elemente oder Schritte ausschließen und unbestimmte Artikel wie "ein" oder "eine" keine Vielzahl ausschließen.

Ferner wird darauf hingewiesen, dass Merkmale oder Schritte, die mit Verweis auf eine der vorstehenden Ausführungsformen beschrieben sind, auch in Kombination mit Merkmalen oder Schritten, die mit Verweis auf andere der vorstehenden Ausführungsformen beschrieben sind, verwendet werden können.

Bezugszeichen in den Ansprüchen sind nicht als Beschränkung des Umfangs des durch die Ansprüche definierten Gegenstands zu verstehen.

### Liste der Bezugszeichen

- 1: Beatmungsgerät
- 3: Atemluftanschluss
- 5: Aktorik
- 7: Atemluftstrom
- 9: Sensorik
- 11: Messdaten
- 13: Steuereinheit
- 15: Atemapparat
- 17: Atemwege
- 19: Lunge
- 21: Prozessor
- 23: Speicher
- 25: Steuersignal
- 27: druckabhängiger Verlauf
- 28: zeitabhängiger Verlauf
- 29: volumenabhängiger Verlauf
- p: Druck
- p_{c_lu}: Lungenverschlussdruck
- p_{o_aw}: Atemwegöffnungsdruck
- p_{o_lu}: Lungenöffnungsdruck
- p_{E}: Ausatmungsdruck
- p_{I}: Einatmungsdruck
- pCO₂: Kohlendioxidpartialdruck
- t: Zeit, Beatmungsdauer
- E: Ausatmungsphase
- I: Einatmungsphase
- M: Verfahren
- Q: Volumenstrom
- S1: Erzeugen eines Steuersignals
- S2: Empfangen von Messdaten
- S3: Bestimmen eines Öffnungs- und/oder Verschlussdrucks
- S4: Bestimmen eines oder mehrerer Sollwerte
- S5: Verwenden eines oder mehrerer Sollwerte
- V: Volumen
- V_{c_lu}: Lungenverschlussvolumen

## Patentansprüche

1. Steuereinheit (13) für ein Beatmungsgerät (1), wobei das Beatmungsgerät (1) umfasst:
einen Atemluftanschluss (3), an den der Atemapparat (15) eines Patienten angeschlossen ist, sodass eine Beatmung des Patienten mit Atemluft möglich ist;
eine Aktorik (5) zum Bereitstellen eines Atemluftstroms (7) am Atemluftanschluss (3);
eine Sensorik (9) zum Erzeugen von Messdaten (11) bezüglich der Beatmung;
wobei die Steuereinheit (13) konfiguriert ist, um folgendes Verfahren (M) auszuführen:
Erzeugen (S1) eines Steuersignals (25) zum Steuern der Aktorik (5), sodass ein Beatmungsmanöver durchgeführt wird, bei dem zumindest ein Abschnitt (17, 19) des Atemapparats (15) in einer Einatmungsphase (I) geöffnet und/oder in einer Ausatmungsphase (E) verschlossen wird;
Empfangen (S2) der Messdaten (11), wobei die Messdaten (11) einen druckabhängigen Verlauf (27) einer vom Patienten in der Einatmungsphase (I) eingeatmeten und/oder in der Ausatmungsphase (E) ausgeatmeten Menge (pCO₂) mindestens eines Atemgases abhängig von einem Druck (p, p_{E}, p_{I}) der Atemluft anzeigen;
Bestimmen (S3) eines Öffnungsdrucks (p_{o_aw}, p_{o_lu}), der einem Druck (p, p_{I}) der Atemluft entspricht, bei dem zumindest ein Abschnitt (17, 19) des Atemapparats (15) geöffnet wird, durch Auswerten eines auf die Einatmungsphase (I) bezogenen Abschnitts des druckabhängigen Verlaufs (27), und/oder Bestimmen (S3) eines Verschlussdrucks (p_{c_lu}), der einem Druck (p, p_{E}) der Atemluft entspricht, bei dem zumindest ein Abschnitt (17, 19) des Atemapparats (15) verschlossen wird, durch Auswerten eines auf die Ausatmungsphase (E) bezogenen Abschnitts des druckabhängigen Verlaufs (27).

2. Steuereinheit (13) nach Anspruch 1,
wobei der Öffnungsdruck (p_{o_aw}, p_{o_lu}) umfasst: einen Atemwegöffnungsdruck (p_{o_aw}), der einem Druck (p, p_{I}) der Atemluft entspricht, bei dem die Atemwege (17) des Patienten zumindest teilweise geöffnet werden, und/oder einen Lungenöffnungsdruck (p_{o_lu}), der einem Druck (p, p_{I}) der Atemluft entspricht, bei dem die Lunge (19) des Patienten zumindest teilweise geöffnet wird; und/oder
wobei der Verschlussdruck (p_{c_lu}) einen Lungenverschlussdruck (p_{c_lu}) umfasst, der einem Druck (p, p_{E}) der Atemluft entspricht, bei dem die Lunge (19) des Patienten zumindest teilweise verschlossen wird.

3. Steuereinheit (13) nach einem der vorhergehenden Ansprüche,
wobei das Steuersignal (25) so erzeugt wird, dass ein Einatmungsdruck (p_{I}), der einem Druck (p) der Atemluft in der Einatmungsphase (I) entspricht, einem vorgegebenen Einatmungsdruckverlauf folgt, der zumindest teilweise linear ansteigt, und/oder in mehreren aufeinanderfolgenden Zeitschritten erhöht wird, wobei der Einatmungsdruck (p_{I}) in jedem Zeitschritt höher als im jeweils vorangegangenen Zeitschritt ist.

4. Steuereinheit (13) nach einem der vorhergehenden Ansprüche,
wobei das Steuersignal (25) so erzeugt wird, dass ein Volumenstrom (Q) der Atemluft in der Ausatmungsphase (E) einem vorgegebenen Volumenstromverlauf folgt; und/oder
wobei ein Volumenstrom (Q) der Atemluft in der Ausatmungsphase (E) zwischen 0,02 l/s und 0,20 I/s, bevorzugt zwischen 0,05 l/s und 0,15 I/s, besonders bevorzugt bei 0,10 l/s, liegt.

5. Steuereinheit (13) nach einem der vorhergehenden Ansprüche,
wobei das Steuersignal (25) so erzeugt wird, dass ein Ausatmungsdruck (p_{E}), der einem Druck (p) der Atemluft in der Ausatmungsphase (E) entspricht, einem vorgegebenen Ausatmungsdruckverlauf folgt, der zumindest teilweise linear abfällt, und/oder in mehreren aufeinanderfolgenden Zeitschritten verringert wird, wobei der Ausatmungsdruck (p_{E}) in jedem Zeitschritt niedriger als im jeweils vorangegangenen Zeitschritt ist.

6. Steuereinheit (13) nach einem der vorhergehenden Ansprüche,
wobei das Bestimmen (S3) des Öffnungsdrucks (p_{o_aw}, p_{o_lu}) und/oder des Verschlussdrucks (p_{c_lu}) umfasst:
Erkennen eines charakteristischen Knickpunkts im druckabhängigen Verlauf (27);
Bestimmen eines dem charakteristischen Knickpunkt zugeordneten Drucks (p, p_{E}, p_{I}) der Atemluft, um den Öffnungsdruck (p_{o_aw}, p_{o_lu}) und/oder den Verschlussdruck (p_{c_lu}) zu bestimmen.

7. Steuereinheit (13) nach Anspruch 6,
wobei der charakteristische Knickpunkt anhand einer charakteristischen betragsmäßigen Verringerung einer durchschnittlichen Steigung des druckabhängigen Verlaufs (27) mit zunehmender Beatmungsdauer (t) erkannt wird.

8. Steuereinheit (13) nach einem der vorhergehenden Ansprüche,
wobei die Einatmungsphase (I) und die Ausatmungsphase (E) aufeinanderfolgende Phasen eines einzelnen Atemzugs sind; und/oder
wobei zum Bestimmen (S3) des Öffnungsdrucks (p_{o_aw}, p_{o_lu}) ein Abschnitt des druckabhängigen Verlaufs (27) in der ersten Hälfte oder im ersten Drittel der Einatmungsphase (I) ausgewertet wird und/oder zum Bestimmen (S3) des Verschlussdrucks (p_{c_lu}) ein Abschnitt des druckabhängigen Verlaufs (27) im letzten Drittel der Ausatmungsphase (E) ausgewertet wird.

9. Steuereinheit (13) nach einem der vorhergehenden Ansprüche,
wobei die Messdaten (11) ferner einen volumenabhängigen Verlauf (29) der vom Patienten in der Einatmungsphase (I) eingeatmeten und/oder in der Ausatmungsphase (E) ausgeatmeten Menge (pCO₂) des mindestens einen Atemgases abhängig von einem Volumen (V) der Atemluft anzeigen, wobei der Öffnungsdruck (p_{o_aw}, p_{o_lu}) ferner durch Auswerten eines auf die Einatmungsphase (I) bezogenen Abschnitts des volumenabhängigen Verlaufs (29) bestimmt wird und/oder der Verschlussdruck (p_{c_lu}) ferner durch Auswerten eines auf die Ausatmungsphase (E) bezogenen Abschnitts des volumenabhängigen Verlaufs (29) bestimmt wird.

10. Steuereinheit (13) nach einem der vorhergehenden Ansprüche,
wobei die Messdaten (11) ferner einen zeitabhängigen Verlauf (28) eines vom Patienten in der Einatmungsphase (I) eingeatmeten und/oder in der Ausatmungsphase (E) ausgeatmeten Volumenstroms (Q) der Atemluft abhängig von einer Beatmungsdauer (t) anzeigen, wobei der Öffnungsdruck (p_{o_aw}, p_{o_lu}) ferner durch Auswerten eines auf die Einatmungsphase (I) bezogenen Abschnitts des zeitabhängigen Verlaufs (28) bestimmt wird und/oder der Verschlussdruck (p_{c_lu}) ferner durch Auswerten eines auf die Ausatmungsphase (E) bezogenen Abschnitts des zeitabhängigen Verlaufs (28) bestimmt wird.

11. Steuereinheit (13) nach einem der vorhergehenden Ansprüche,
wobei das Verfahren (M) ferner umfasst:
Bestimmen (S4) mindestens eines Sollwerts für den Druck (p, p_{E}, p_{I}) der Atemluft unter Berücksichtigung des Öffnungsdrucks (p_{o_aw}, p_{o_lu}) und/oder des Verschlussdrucks (p_{c_lu});
Verwenden (S5) des mindestens einen Sollwerts zum Steuern der Aktorik (5).

12. Steuereinheit (13) nach einem der vorhergehenden Ansprüche,
wobei die Messdaten (11) Werte für mindestens eine der folgenden Größen umfassen und/oder auf Werten für mindestens eine der folgenden Größen basieren: die vom Patienten in der Einatmungsphase (I) eingeatmete und/oder in der Ausatmungsphase (E) ausgeatmete Menge (pCO₂) des mindestens einen Atemgases, den Druck (p, p_{E}, p_{I}) der Atemluft, ein Volumen (V) der Atemluft, einen Volumenstrom (Q) der Atemluft.

13. Beatmungsgerät (1), umfassend:
einen Atemluftanschluss (3) zum Anschließen des Atemapparats (15) eines Patienten, sodass eine Beatmung des Patienten mit Atemluft möglich ist;
eine Aktorik (5) zum Bereitstellen eines Atemluftstroms (7) am Atemluftanschluss (3);
eine Sensorik (9) zum Erzeugen von Messdaten (11) bezüglich der Beatmung;
eine Steuereinheit (13) nach einem der vorhergehenden Ansprüche.

14. Computerprogramm zum Betreiben des Beatmungsgeräts (1) nach Anspruch 13, wobei das Computerprogramm Befehle umfasst, die die Steuereinheit (13) beim Ausführen des Computerprogramms durch die Steuereinheit (13) veranlassen, folgendes Verfahren (M) auszuführen:
Erzeugen (S1) eines Steuersignals (25) zum Steuern der Aktorik (5), sodass ein Beatmungsmanöver durchgeführt wird, bei dem zumindest ein Abschnitt (17, 19) des Atemapparats (15) in einer Einatmungsphase (I) geöffnet und/oder in einer Ausatmungsphase (E) verschlossen wird;
Empfangen (S2) der Messdaten (11), wobei die Messdaten (11) einen druckabhängigen Verlauf (27) einer vom Patienten in der Einatmungsphase (I) eingeatmeten und/oder in der Ausatmungsphase (E) ausgeatmeten Menge (pCO₂) mindestens eines Atemgases abhängig von einem Druck (p, p_{E}, p_{I}) der Atemluft anzeigen;
Bestimmen (S3) eines Öffnungsdrucks (p_{o_aw}, p_{o_lu}), der einem Druck (p, p_{I}) der Atemluft entspricht, bei dem zumindest ein Abschnitt (17, 19) des Atemapparats (15) geöffnet wird, durch Auswerten eines auf die Einatmungsphase (I) bezogenen Abschnitts des druckabhängigen Verlaufs (27), und/oder Bestimmen (S3) eines Verschlussdrucks (p_{c_lu}), der einem Druck (p, p_{E}) der Atemluft entspricht, bei dem zumindest ein Abschnitt (17, 19) des Atemapparats (15) verschlossen wird, durch Auswerten eines auf die Ausatmungsphase (E) bezogenen Abschnitts des druckabhängigen Verlaufs (27).

15. Computerlesbares Medium, auf dem das Computerprogramm nach Anspruch 14 gespeichert ist.
